## Europäisches Patentamt
## European Patent Office
## Office européen des brevets

(19)

(11) Publication number: **0 000 969**
**B1**

(12) # EUROPEAN PATENT SPECIFICATION

(45) Date of publication of patent specification: **02.12.81**

(51) Int. Cl.³: **A 61 F 13/16, A 41 B 9/12**

(21) Application number: **78200142.4**

(22) Date of filing: **18.08.78**

(54) Absorbent brief.

(30) Priority: **30.08.77 US 829035**

(43) Date of publication of application:
**07.03.79 Bulletin 79/5**

(45) Publication of the grant of the European patent:
**02.12.81 Bulletin 81/48**

(84) Designated Contracting States:
**CH DE FR GB LU NL SE**

(56) References cited:
**FR - A - 2 242 043**
**FR - A - 2 345 955**
**US - A - 3 452 753**
**US - A - 3 860 003**

(73) Proprietor: **THE PROCTER & GAMBLE COMPANY**
**301 East Sixth Street**
**Cincinnati Ohio 45201 (US)**

(72) Inventor: **Strickland, Danny Leroy**
**4353 Springdale Road**
**Cincinnati Ohio 45239 (US)**
Inventor: **Visscher, Ronald Bosman**
**11630 Morrocco Court**
**Cincinnati Ohio 45240 (US)**

(74) Representative: **Munro, Hamish David et al,**
**PROCTER & GAMBLE EUROPEAN TECHNICAL**
**CENTER Temselaan 100**
**B-1820 Strombeek-Bever (BE)**

Courier Press, Leamington Spa, England.

Absorbent brief

This invention relates generally to improvements in a disposable absorbent brief intended to be used to receive or tending to receive discharge from the body, and which functions as a disposable diaper, incontinent pad, or the like designed to be worn on the body and having a contractable thigh-encircling portion which conforms to the contours of the body and permits movement of the body while maintaining a seal with the body in motion. This invention further relates to means to secure such a brief to the body of the wearer and more particularly to means adapting the thigh-encircling portions of said garment to an adult leg configuration.

Absorptive devices such as disposable incontinent briefs are well-known in the art. These devices are used to absorb liquid from the human body and retain that liquid until the garment can be disposed of. Present disposable incontinent briefs are frequently flat composite sheets which are fitted to a wearer in the flat state or incorporate geometric folding to achieve a suitable body shape. A major in-use problem with such prior art articles is that gaps between the brief and the wearer's legs tend to develop due to the semi-rigid nature of the absorbent body, especially after the brief has been worn for some time, or during a period of activity when the garment is able to flex or shift on the wearer's body. These gaps permit leakage from a disposable garment, thereby creating damp outer clothing or bedding around the wearer.

Although these problems have been alleviated somewhat in the case of infant's diapers, the fitting of such a garment to an adult presents problems not found in the creation of a garment suitable for an infant.

First, since the legs of an infant characteristically form a substantial angle with the lateral line of the trunk of the body, it has been observed that a diaper having straight-line elastics such as those described in U.S. Patent 3,860,003 issued to Buell on January 14, 1975, can be secured to the infant's body by a single securement means on either side of the diaper. This is true because a circle drawn around the top portion of the diaper on the baby's body and a circle drawn around the thigh seal portion very nearly intersect at one tangent point, so that a single securement means placed at that point will effectively secure the waist and the thigh portions of the diaper simultaneously. However, in an adult, whose legs in the normal position are generally substantially parallel to each other and to the lateral line of the trunk of the wearer's body, a circle drawn at the edge of the garment about the wearer's thigh and substantially perpendicular to the wearer's thigh as defined hereinafter does not at any point approach a second circle drawn around the top portion of the garment secured about the wearer's waist or trunk. Thus, unlike prior art devices primarily designed to fit an infant, a single securement means on each side of the article will not serve to conform the garment to an adult wearer's waist while effectively securing the garment against leakage at the thigh area.

Second, as also revealed in the aforementioned Buell patent, it is desirable to construct an incontinent brief with elastics which are parallel to each other, and which lie along each edge of the crotch. This simplifies the manufacture of an incontinent brief, since the elastic can be applied in the machine direction when the article is manufactured. Brief elastics having a substantially straight-line contractable dimension have advantages in use as well. A substantially straight-line elastic member in the region of the crotch allows a single article to contract substantially along this dimension, so that the differing crotches of various individuals are accommodated to provide a snug fit of the article in the crotch area of the wearer. (This development of the prior art is in contrast to a great number of similar articles, such as conventional underwear, deploying elastic members which have no substantial straight-line element, and which generally conform around a cut-out in the brief.) However, a corresponding problem in prior art structures employing parallel straight-line elastic members having a substantially longitudinal contractable dimension and a single securement means to secure the waistband and legband portions, especially in an adult garment where the crotch width must necessarily be much less than the width of the portion of the garment which wraps around the trunk of the wearer, is that one cannot draw the functional ends of the straight-line elastic member effectively together around the thigh of the wearer to form a seal which is substantially perpendicular (as defined hereinafter) to the thigh bone of the wearer without unduly compromising the fit of the waistband portion about the wearer's trunk. As pointed out above, a seal which is not substantially perpendicular to the thigh bone of the wearer will not adequately contain the material which is to be absorbed or held by the brief.

In summary, devices can be found in the prior art which have straight-line elastic members on either side of the crotch; prior art structures having roughly the same can be found wherein a plurality of fasteners are used on either side of a garment to attach it to the body of a wearer. However, prior art is not available to solve the specific problems which are encountered when straight-line elastic members are used in a garment which requires a plurality of fastening means on either side of the body of the wearer in order to seal the garment to the thigh while providing a fit to the trunk of the wearer as well.

## Summary of the Invention

The present invention lies in the provision of an improved thigh seal in a disposable absorbent brief particularly adapted to an adult wearer, as defined hereinafter, and having substantially straight-line elastic portions in the crotch area thereof. This seal, which is not elasticized around its entire perimeter when the brief is in use, is facilitated by placement of the anchored portion of a thigh securement means on an anchoring region of the brief surface located intermediate two line segments lying respectively on the transversely outward legs of two angles having their apexes at one functional endpoint of the brief elastic, the first angle being about 90 degrees transversely outward with respect to the elastic member, and the second angle being about 140 degrees transversely outward with respect to the same elastic member, said anchoring portion of the brief surface being bordered on its third side by the edge of the brief at a lower part of the trunk-encircling portion.

When the brief is donned by encircling the thigh of the wearer with the lower part of one trunk-encircling portion of the brief, the user-actuated portion of a securement means which is already anchored on the anchoring portion as above defined is further secured to the lower part of the second trunk-encircling portion of the brief in a position effective to achieve the desired seal. The site of attachment of the user-actuated portion of the lower securement means is somewhere on the backsheet of the second trunk-encircling portion, the exact site depending on the particular thigh, trunk, and crotch dimensions of the wearer.

As a result of the critical placement of the anchored portion of the thigh securement means on the indicated segment of the brief, a seal is generally defined by the following structures: the elastic member; a portion of the brief material lying along a tensioned line between the functional endpoint of the elastic member which defines the apexes described above and the anchored portion of the thigh securement means; and the user-actuated portion of the thigh securement means. If the user-actuated portion of the thigh securement means is attached to the brief transversely outward of the elastic member, the portion of brief material between that point of attachment and the second functional end of the elastic forms a portion of the seal as well. It is of the essence in this invention that the elements of the seal defined above lie substantially in a plane perpendicular, as defined hereinafter, to the thigh bone of the wearer when the brief is in use.

It should be noted that while the brief requires further means to conform it about the trunk of the wearer, such means are known in the art and are not within the scope of this invention.

## Objects of the Invention

The objects of this invention are to solve the problems noted in the prior art, as well as to provide an incontinent brief with an improved thigh seal, suitable for use by adults and capable of being worn under the clothing of an adult as well as by an adult who is bed-ridden. Further objects of the invention will become apparent from the description following.

## Brief Description of the Drawings

Exemplary embodiments of the invention are described hereinafter with reference to the accompanying drawings in which:

Figure 1 is a plan view of a preferred embodiment of the invention with its elastic members in an extended condition and the wearer contacting portion of the brief facing the viewer.

Figure 2 is a plan view of the device of Figure 1 illustrating a preferred embodiment of the invention, with the elastic in an unstressed condition.

Figure 3 is a plan view of an alternate embodiment of the invention, with its elastic members in an extended condition, and with its wearer contacting portion facing the viewer.

## Detailed Description of the Invention

For the purposes of the specification, an "adult" is defined as any person having thigh bones which are oriented substantially parallel to the lateral line of the body during a majority of the wearer's activities. This distinguishes an "adult" from an infant, and particularly from an infant who has not yet learned to walk, for the legs of such an infant usually form a substantial angle with respect to the lateral line of its body during a majority of the wearer's activities. Thus, an "adult" as defined herein may include youths and teenagers who have leg orientations similar to those of a mature person.

A plane which is "substantially perpendicular" to the thigh-bone of the wearer is defined herein as a plane forming a plane angle of between about 0 degrees and about 40 degrees with respect to a plane which is geometrically perpendicular to the thigh-bone of the wearer.

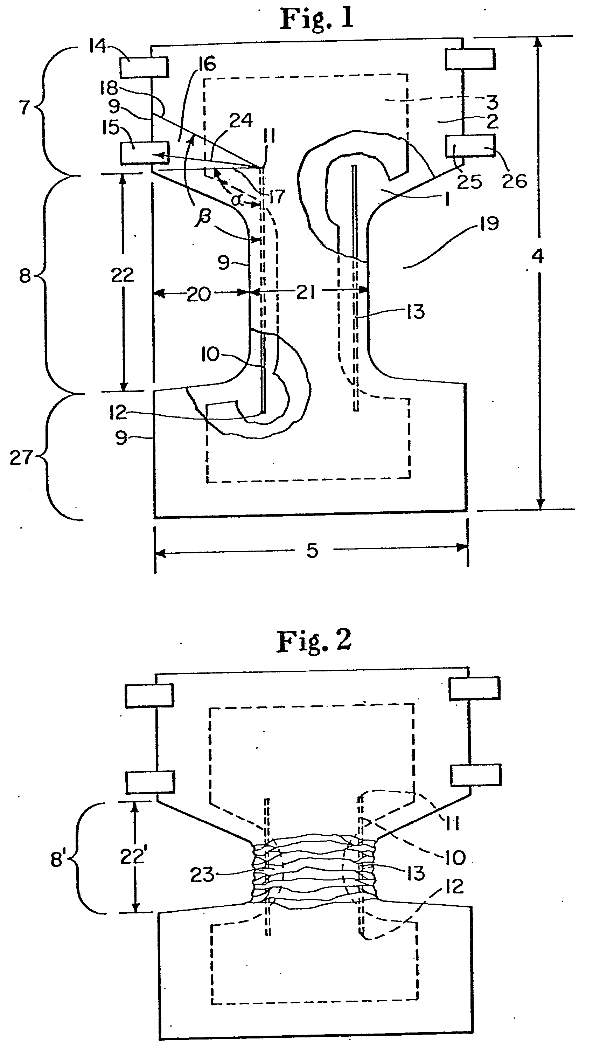

Figures 1 and 2 represent a particularly preferred embodiment of the present invention having side notches which receive the legs of the wearer in order to provide a brief having a well-tailored contour when it is worn.

The absorbent brief forming the present invention is comprised generally of moisture-impervious backsheet 1, moisture-pervious topsheet 2, and moisture-absorbent member 3 therebetween. The backsheet 1 and topsheet 2 are preferably secured to one another about absorbent member 3 at the periphery of the article which is generally indicated by 9.

The dimensions of the brief of Figure 1 are more particularly described by a longitudinal dimension 4, a transverse dimension 5, a crotch portion 8 having a crotch length 22, a crotch width 21, a cut-out width 20, and trunk-encircling portions 7 and 27. It is to be understood that the brief may be worn with either trunk-encircling portion 7 or trunk-encircling portion 27 forward, although in the preferred mode of use the trunk-encircling portion 7 forms the rear portion of the garment when it is worn. Crotch portion 8 of the article is provided with a plurality of elastic members 10 preferably one on each side of the wearer's crotch, and generally disposed near the edge of the garment. Each elastic member 10 has endpoints 11 and 12, hereinafter referred to as "functional endpoints" because it is appreciated that an end portion of elastic member 10 may be provided which is unable to gather or shirr the garment because it is either stiffened or unadhered to the article.

Absorbent member 3 of the brief can be made of any of the absorbent materials known to those of ordinary skill in the bandage art. For example: a multiplicity of plies of creped cellulose wadding; fluffed cellulosic fibers or air-laid wood pulp fibers sometimes known as air-felt fibers or other absorbent material. A particularly preferred absorbent member 3 may be constructed following the teachings of U.S. Patent 3,860,003. While in the embodiment of Figure 1 absorbent member 3 lies generally between elastic members 10 in the crotch portion of the brief it is to be appreciated that this is not a requirement of the present invention, so long as elastic members 10 are able to contract the brief.

Moisture-impervious backsheet 1 is preferably a flexible moisture-impermeable sheet comprised of a low density, opaque polyethylene web having a thickness of about 0.025 mm, such as the backsheet employed in the aforementioned USP 3860003. In the embodiment illustrated in Figure 1, the backsheet 1 has a rectangular configuration with leg cut-out areas 19 deviating from the rectangular configuration. In this embodiment the backsheet 1 extends beyond the periphery of the absorbent body around the entire periphery thereof. Brief side portion 9 defined generally by the periphery of the backsheet extending beyond absorbent member 3, is roughtly 100 mm wide in the trunk-encircling portions 7 and 27 and approximately 37.5 mm to 50 mm wide in the crotch portion 8 of the brief.

Topsheet 2 is coextensive in area with backsheet 1. One preferred embodiment of topsheet 2 is shown and described in U.S. Patent 3,929,135 issued to Hugh Thompson on December 30, 1975. However, it is to be understood that the invention does not require a particular type of topsheet, or any topsheet at all.

The elastic member 10 is operatively associated with crotch portion 8 adjacent to brief side portion 9 in an elastically contractable condition so that in a normally unrestrained configuration, the elastic member 10 effectively contracts or gathers the crotch material to provide an elastic retraction line 13 colinear with the material of elastic member 10. Attachment of the elastic members 10 to achieve this result is also described in the aforementioned USP 3860003.

A comparison between Figures 1 and 2 will illustrate the function of the elastic members 10 on either side of the crotch to adjust the length of the crotch to fit the body of a wearer. In Figure 1, wherein the elastic members are stretched so that the entire garment lies substantially in a plane, crotch portion 8 has a maximized crotch length dimension 22. On the other hand, in Figure 2, where the elastic is allowed to relax insofar as relaxation is permitted by the material of the brief, the crotch length dimension 22' is roughly one-half as large as the identical dimension in Figure 1. The exact variance between the dimensions 22 and 22' will depend on how the side-notch and crotch are dimensioned in a particular garment. As depicted by the reference numeral 23, the material in the crotch is gathered in Figure 2, but the width 21 of the crotch is substantially unchanged between the two Figures. It has been found that when the crotch is elastically contractable in the longitudinal direction without substantial transverse contraction, the brief is well-suited to accommodate a wide range of sizes without the need for measurements or mechanical adjustments.

The effective length of elastic member 10 in its stretched condition is that length available to contract. The extremities of this effective length of elastic are defined by functional endpoints 11 and 12. These functional endpoints 11 and 12 may be distinguished from the actual endpoints of the elastic, for it is apparent that portions of the elastic may be so secured to the brief that they are unable to contract, or loose ends of the elastic may be allowed which do not contribute to the contraction of the brief.

Upper securement means 14 may be provided by any of a number of means well-known in the art, such as tapes, an elastic band to which both ends of the brief are attached (analogous to the waistband of conventional underwear), snaps, pins, and so forth. Likewise, it is to be appreciated that lower securement means 15 can be any securement means which can be used to attach two portions of the garment together while it is being worn. One particularly preferred fastening means is the tape fastening structure generally disclosed in U.S. Patent 3,848,594 issued to Buell on November 19, 1974.

A critical feature of the present invention is the placement of lower securement means 15. Lower securement means 15 is anchored by a manufacturer's joint at its anchored portion 25 to the brief within anchoring region 16, which is defined by the edge portion of the brief 9 and by line segments 17 and 18. First line segment 17 is bounded at its transversely inward end by functional endpoint 11 of the elastic member 10, and extends transversely outward from functional endpoint 11 in the plane of the brief at an angle $\alpha$ with respect to elastic member 10. Second line segment 18 similarly is bounded by functional endpoint 11 and extends transversely outward to the edge portion 9 of the brief in the plane of the brief at an angle $\beta$ with respect to elastic member 10. The angle $\alpha$ is preferably about 90 degrees, and the angle $\beta$ is preferably about 140 degrees when the elastics are located within 37.5 mm of the lateral edge of the crotch portion of the diaper along a majority of their length. Particularly preferred values of $\alpha$ and $\beta$ are about 116 degrees and about 125 degrees, respectively.

When lower securement means 15 is anchored in region 16, securement vector 24, a tension vector, defines a portion of the brief between lower securement means 15 and functional endpoint 11 which lies approximately in a plane which is substantially perpendicular to the thigh of the wearer when the brief is worn, forming a part of the thigh seal.

To attach the brief to the body of the wearer, the brief is placed around the crotch and trunk portions of the wearer, upper securement means 14 is brought laterally around the trunk of the wearer and secured to the other side of the brief somewhere along the upper part of trunk-encircling portion 27. Similarly, lower securement means 15 is secured to the lower part of trunk-encircling portion 27. The points of securement of upper and lower securement means 14 and 15 are thus variable, depending on the particular body dimensions of the wearer in comparison to the dimensions of the brief.

When the garment is donned and attached as described above, elastic member 10 lies along the inner portion of the thigh of the wearer, and securement vector 24 lies along a second portion of the wearer's thigh. If the point of attachment of the user-actuated portion 26 of lower securement means 15 on trunk-encircling portion 27 is transversely inward of the line defined by elastic member 10, securement vector 24 overlies that line and is held in that overlying relation by circumferential tension to complete the seal. On the other hand, if the point of attachment of the user-actuated portion 26 of lower securement means 15 is transversely outward of a line defined by elastic member 10, the material of the garment spanning the space between said point of attachment and functional endpoint 12 of elastic member 10 completes the seal. When the brief is worn, these portions of the seal are all aproximately in a plane which is substantially perpendicular to the thigh-bone of the wearer, and form a substantially unbroken seal around the thigh of the wearer. This thigh seal is resilient due to the action of elastic member 10, which contracts along elastic retraction line 13 to conform the seal to the thigh of the wearer. Thus, this structure satisfies the objects of this invention to provide a thigh seal which lies approximately in a plane which is substantially perpendicular to the thigh-bone of the wearer.

Briefs made according to this preferred embodiment of the invention can conveniently be made in small, medium and large sizes to accommodate the proportions of a wide number of consumers. A medium-sized brief adapted to fit the ordinary range of adult wearers having hip circumferential measurements in the range of 813 mm—1118 mm can be accommodated by a brief having a functional stretched elastic length of 203—229 mm, a longitudinal dimension 4 of approximately 838 mm, a transverse dimension 5 of approximately 635 mm, a crotch width 21 of approximately 241 mm, and spacing between elastic members 10 across the crotch of approximately 203 mm. To accommodate the dimensions of a small adult or teenage wearer, a brief may conveniently be dimensioned with a functional stretched elastic length of 203—229 mm, a longitudinal dimension 4 of approximately 711 mm, a transverse dimension 5 of approximately 457 mm, a crotch width 21 of approximately 178 mm, and spacing between elastic members 10 of approximately 178 mm, and spacing between elastic members 10 of approximately 152 mm across the crotch. A brief for large adults may conveniently have the following dimensions: a functional stretched elastic length of 203—229 mm, a longitudinal dimension 4 of approximately 1016 mm, a transverse dimension 5 of approximately 787 mm, a crotch width 21 of approximately 241 mm, and a spacing between elastic members 10 of approximately 203 mm. While it is to be appreciated that the invention is not limited to embodiments having these dimensions, it has been found that these three brief sizes will accommodate a selection of wearers ranging from adolescents to rather large adults.

When a brief constructed in accordance with the above "medium" dimensions was worn by a particular female model, it was found that the separation between an upper tape, located with its center about an inch below the top of the brief, and the center of the thigh securement tape, was approximately 140 mm when an acceptable thigh seal was formed. When the thigh seal was optimized, the tape separation for this wearer ranged between 150 mm and 203 mm. Within this range, the angle of the plane of the seal with respect to a plane perpendicular to the thigh-bone varied between about 20 degrees and about 30 degrees. When the angle of the plane of the seal exceeded about 40 degrees, the seal was no longer adequate. These figures for a single wearer are not intended to be descriptive of the entire range of brief wearers, but they illustrate that a brief designed for adult wear must have independent means to respectively secure the garment at the thigh and at the waist.

The optimum angle of the plane of the seal with respect to a plane perpendicular to the thigh of the wearer is an accommodation between the frequently conflicting goals of providing an effective thigh seal and providing a desirable overall fit of the brief to the wearer's body. The ideal angle from a sealing standpoint is nearly 0 degrees, at which point the circumference of the seal is minimized. The ideal angle from a fit standpoint would be much larger for many persons to form an ideal brief-shaped garment. The angles encompassed by the definition of "substantially perpendicular" in this specification represent the middle ground of configurations which will seal adequately while providing an acceptable fit.

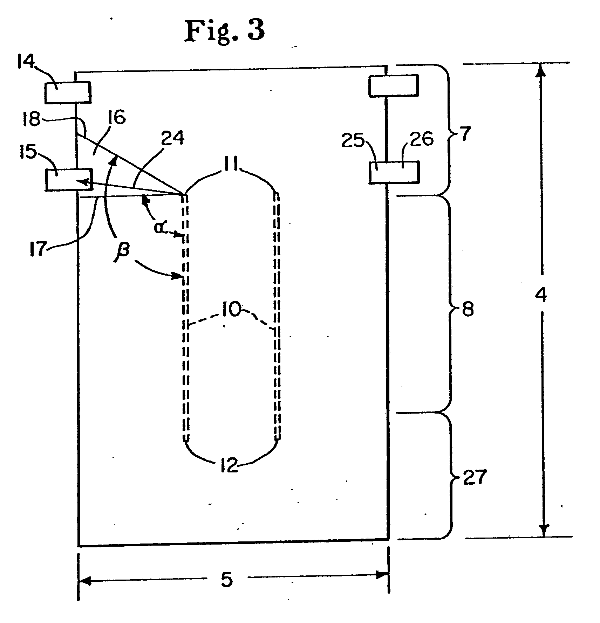

Figure 3 shows an alternate, but less preferred embodiment of the invention wherein cut-out 19 is attenuated or eliminated from the brief. It will be appreciated that this embodiment will only work if portions of the brief material transversely outward of elastic member 10 will gather in response to retraction of elastic members 10 along elastic retraction lines 13 and will fold when the brief is worn so that the seal as described for the preferred embodiment of the invention will not be hindered by the presence of this material. It will further be appreciated that the general overall shape of the brief may be modified substantially in other ways from the preferred embodiment without departing from the scope of the invention, which is defined by the claims. The most preferred values of $\alpha$ and $\beta$ for this embodiment are about 125 degrees and about 132 degrees, respectively when the elastics are located within approximately 216 mm of the lateral edge of the crotch portion of the diaper along a majority of their length. As this distance is decreased, the included angle $(\beta - \alpha)$ generally increases.

## Claims

1. A generally rectangular disposable absorbent brief comprising first and second trunk encircling portions (7, 27) having laterally extending end edges defining the longitudinal dimension (4) of the brief, a crotch portion (8) disposed between the trunk encircling portions (7, 27), the side edges of said trunk encircling and crotch portions defining the lateral dimension (5) of the brief, a pair of elastic strips (10) each secured along opposite sides of the crotch portion (8), the strips (10) extending through the crotch portion (8) in a line terminating at functional end points (11, 12), and being respectively disposed along retraction lines (13) extending substantially perpendicularly to the laterally extending end edges of the trunk-encircling portions (7, 27), and a pair of adhesive tape fasteners (14) to secure the trunk encircling portions (7, 27) to each other about the wearer's trunk, characterised in that a second pair of tape fasteners (15, 26) are secured to the brief within an anchoring region (16) spaced outwardly from the respective functional end points (11, 12), each of said anchoring regions (16) being defined by two line segments extending laterally outwardly from said functional end points to the adjacent side edge of the brief, the first of said line segments subtending an angle of at least 90° as measured from the elastic strip 10, the second of said line segments subtending an angle of no more than 140° as measured from the same elastic strip (10) whereby, in use, said second tape fastener, said elastic strip and material of said brief intermediate the functional end points of said elastic strip and said second tape fastener combine to form an elastic seal about the wearer's thigh, the plane of the seal being substantially perpendicular to the longitudinal axis of the wearer's thigh, the seal being formed independently of the securement of the trunk encircling portions (7, 27).

2. A disposable absorbent brief according to Claim 1 characterised in that the crotch portion is of reduced width compared to the trunk encircling portions and in that the strips are spaced within 37.5 mm of the respective side edges of the crotch portion along a majority of its length.

3. A disposable absorbent brief according to Claim 2 characterised in that the shape of the crotch portion is defined by substantially trapezoidal inwardly tapered cut-outs in the longer sides of the brief.

4. A disposable absorbent brief according to Claim 3 characterised in that said first and second line segments respectively subtend angles of 116° and 125° as measured from the elastic strips at their functional endpoints.

5. A disposable absorbent brief according to Claim 1 characterised in that the elastic strips are located within 216 mm of the respective side edges of the crotch portion.

6. A disposable absorbent brief according to any one of the preceding claims characterised in that the second tape fastener is an adhesive tape anchored at one end to one trunk encircling portion and adapted in use to engage the other corresponding trunk encircling portion when the brief is applied to the wearer.

## Revendications

1. Bandage absorbant à jeter après usage, de forme générale rectangulaire, comprenant une première et une seconde partie d'entourage de tronc (7, 27) pourvues de bords extrêmes s'étendant latéralement et définissant la dimension longitudinale (4) du bandage; une partie de fourche (8) disposée entre les parties d'entourage de tronc (7, 27), les bords latéraux desdites parties d'entourage de tronc et de fourche définissant la dimension latérale (5) du bandage; deux bandes élastiques (10) chacune fixées le long de côtés opposés de la partie de fourche (8), lesdites bandes (10) s'étendant au travers de la partie de fourche (8) suivant une ligne se terminant en des points limites fonctionnels (11, 12), et étant respectivement disposées le long de lignes de contraction (13) s'étendant essentiellement perpendiculairement aux bords limites, qui s'étendent latéralement, des parties d'entourage de tronc (7, 27); ainsi que deux attaches à ruban adhésif (14) destinées à fixer les parties d'entourage de tronc (7, 27) l'une sur l'autre autour du tronc de l'utilisateur, caractérisé en ce qu'il est prévu une seconde paire d'attaches à ruban (15), qui sont fixées sur le bandage dans une zone d'ancrage (16) espacée extérieurement des points limites fonctionnels respectifs (11, 12), chacune des zones d'ancrage (16) étant définie par deux segments linéaires (17, 18) s'étendant latéralement vers l'extérieur depuis lesdits points limites fonctionnels jusqu'au bord latéral adjacent du bandage, le premier (17) desdits segments linéaires sous-tendant un angle d'au moins 90°, mesuré à partir de la bande élastique (10), le second (18) desdits segments linéaires sous-tendant un angle non supérieur à 140°, mesuré à partir de la même bande élastique (10) de façon que, en service, ladite seconde attache à ruban, ladite bande élastique et la matière dudit bandage qui est située entre les points limites fonctionnels de ladite bande élastique et ladite seconde attache à ruban coopèrent pour former un joint élastique d'étanchéité autour de la cuisse de l'utilisateur, le plan dudit joint d'étanchéité étant essentiellement perpendiculaire à l'axe longitudinal de la cuisse de l'utilisateur et le joint étanche étant formé indépendamment de la fixation des parties d'entourage de tronc (7, 27).

2. Bandage absorbant à jeter après usage selon la revendication 1, caractérisé en ce que la partie de fourche a une largeur réduite par comparaison aux parties d'entourage de tronc et en ce que les bandes sont espacées de moins de 37,5 mm des bords latéraux respectifs de la partie de fourche sur une grande partie de leur longueur.

3. Bandage absorbant à jeter après usage selon la revendication 2, caractérisé en ce que la forme de la partie de fourche est définie par des échancrures de forme essentiellement trapézoïdale et réduisant de largeur vers l'intérieur, qui sont ménagées dans les côtés longs du bandage.

4. Bandage absorbant à jeter après usage selon l'une des revendications 1 à 3, caractérisé en ce que lesdits premier et second segments linéaires sous-tendent respectivement les angles de 116 degrés et 125 degrés, ces angles étant mesurés à partir des bandes élastiques en leurs points limites fonctionnels.

5. Bandage absorbant à jeter après usage selon l'une des revendications 1 à 4, caractérisé en ce que les bandes élastiques sont placées à moins de 216 mm des bords latéraux respectifs de la partie de fourche.

6. Bandage absorbant à jeter après usage selon l'une quelconque des revendications 1 à 5, caractérisé en ce que la seconde attache à ruban est un ruban adhésif accroché à une extrémité sur une partie d'entourage de tronc et agencé, en service, pour s'accrocher sur l'autre partie d'entourage de tronc correspondante quand le bandage est mis en place sur l'utilisateur.

**Patentansprüche**

1. Praktisch rechteckige, zum Wegwerfen vorgesehene Windelhose mit einem ersten und einem zweiten, zum Einschliessen des Rumpfes vorgesehenen Teil (7, 27) mit in Querrichtung verlaufenden Kanten, welche die Längsabmessung der Windelhose bestimmen, mit einem zwischen den zum Einschliessen des Rumpfes vorgesehenen Teilen (7, 27) angeordneten und zum Einlegen zwischen den Oberschenkeln vorgesehenen Teil (8), wobei die seitlichen Kanten der zum Einschliessen des Rumpfes vorgesehenen Teile und des zum Einlegen zwischen die Oberschenkel vorgesehenen Teils die seitliche Abmessung (5) der Windelhose bestimmen, sowie mit einem Paar elastischer Bänder (10), von denen jedes längs gegenüberliegender Seiten des zum Einlegen zwischen den Oberschenkeln vorgesehenen Teils (8) befestigt ist, welche Bänder (10) geradlinig durch den zum Einlegen zwischen den Oberschenkeln vorgesehenen Teil (8) verlaufen und in funktionellen Endpunkten (11, 12) enden und längs Rückziehlinien (13) angeordnet sind, die praktisch quer zu den in der Querrichtung verlaufenden Endkanten der zum Einschliessen des Rumpfes vorgesehenen Teile (7, 27) verlaufen, und mit einem Paar als Klebstreifen ausgebildeten und zum Befestigen der um den Rumpf des Trägers gelegten Teile (7, 27) aneinander vorgesehene Befestigungselemente (14), dadruch gekennzeichnet, dass an der Windelhose und innerhalb eines Haltebereichs (16), der sich von den zugeordneten funktionellen Endpunkten (11, 12) nach aussen erstreckt, ein zweites Paar bandförmiger Befestigungselemente (15, 26) befestigt ist, wobei jeder dieser Haltebereiche (16) von zwei Teillinien begrenzt ist, die sich von den funktionellen Endpunkten zur benachbarten Seitenkante der Windelhose seitwärts nach aussen erstrecken, wovon die erste dieser Teillinien mit dem elastischen Band (10) einen Winkel von mindestens 90° und die zweite Teillinie mit dem gleichen elastischen Band (10) einen Winkel von nicht mehr als 140° einschliesst, so dass bei der Verwendung dieses zweite bandförmige Befestigungselement, das elastische Band und das Material der Windelhose zwischen den funktionellen Endpunkten des elastischen Bands mit dem zweiten bandförmigen Befestigungsmittel zusammenwirken, um eine elastische Abdichtung um den Oberschenkel des Trägers zu bilden und die Ebene der Abdichtung praktisch quer zur Längsachse des Schenkels des Trägers verläuft und die Abdichtung unabhängig von der Befestigung des zum Einschliessen des Rumpfes vorgesehenen Teils (7, 27) ausgebildet wird.

2. Windelhose nach Anspruch 1, dadurch gekennzeichnet, dass der zum Einlegen zwischen den Schenkeln vorgesehene Teil, verglichen mit dem zum Einschliessen des Rumpfes vorgesehenen Teil, eine verringerte Breite aufweist und dass die Bänder längs des grösseren Teils der Länge des zum Einlegen zwischen den Schenkeln vorgesehenen Teils angeordnet und etwa 37,5 mm von den zugeordneten Seitenkanten beabstandet sind.

3. Windelhose nach Anspruch 2, dadurch gekennzeichnet, dass die Form des zum Einlegen zwischen den Schenkeln vorgesehenen Teils durch praktisch trapezoidal nach innen verengte Ausschnitte in den längeren Seiten der Windelhose begrenzt ist.

4. Windelhose nach Anspruch 3, dadurch gekennzeichnet, dass die erste bzw. zweite Teillinie an den funktionellen Endpunkten mit den elastischen Bändern Winkel von 116° bis 125° einschliessen.

5. Windelhose nach Anspruch 1, dadurch gekennzeichnet, dass die elastischen Bänder etwa 116 mm von der entsprechenden Seitenkante des zum Einlegen zwischen den Schenkeln vorgesehenen Teils angeordnet sind.

6. Windelhose nach irgendeinem der vorstehenden Ansprüche, dadurch gekennzeichnet, dass das zweite bandförmige Befestigungselement ein an seinem einen Ende an einem zum Einschliessen des Rumpfes vorgesehenen Teil befestigtes Klebband ist und beim Gebrauch, wenn die Windelhose dem Träger angelegt ist, am entsprechenden anderen zum Einschliessen des Rumpfes vorgesehenen Teil angeordnet ist.

0 000 969

Fig. 1

Fig. 2

# Fig. 3